# EUROPEAN PATENT SPECIFICATION

(11) **EP 0 448 635 B1**
(45) Date of publication and mention of the grant of the patent: **20.09.1995**
(21) Application number: 90901345.0
(22) Date of filing: 08.12.1989
(51) Int. Cl.: G01N 33/564, G01N 33/53, A61K 39/00

(54) **METHODS AND COMPOSITIONS FOR THE EARLY DETECTION AND TREATMENT OF INSULIN DEPENDENT DIABETES MELLITUS**
VERFAHREN UND ZUSAMMENSETZUNGEN ZUR FRÜHERKENNUNG VON BEHANDLUNG DER INSULINABHÄNGIGEN DIABETES MELLITUS(IDDM)
PROCEDES ET COMPOSITIONS SERVANT A LA DETECTION PRECOCE ET AU TRAITEMENT DU DIABETE SUCRE DEPENDANT DE L'INSULINE

(30) Priority: 13.12.1988 US 283633; 25.10.1989 US 427051
(43) Date of publication of application: 02.10.1991
(73) Proprietor: UNIVERSITY OF FLORIDA, Gainesville, Florida 32611-2037 (US)
(72) Inventor: ATKINSON, Mark, A., Gainesville, FL 32608 (US); MACLAREN, Noel, K., Gainesville, FL 32608 (US)
(74) Representative: Perry, Robert Edward
(86) International application number: US8905570
(87) International publication number: WO9007117

(56) References cited:
- WO-A-89/06968
- JOURNAL OF CLINICAL INVESTIGATION, vol. 79, no. 3, 1987; S. BAEKKESKOV et al., pp. 926-934
- JOURNAL OF IMMUNOLOGY, vol. 137, no. 12, December 1986; I. GERLING et al., pp. 3782-3785
- DIABETES, vol. 37, November 1988; M.A. ATKINSON et al., pp. 1587-1590
- NATIONAL LIBRARY OF MEDICINE (NLM), Medline, file Med 86; C.H. BROGREN et al., acc. no. 86248445
- DIABETES, vol. 36, November 1987; P. VARDI et al., pp. 1286-1291
- NATURE, vol. 298, July 1982; S. BAEKKESKOV et al., pp. 167-169
- SCIENCE, vol. 224, 1984; S. BAEKKESKOV et al., p. 1348
- WEST. JOURNAL OF MEDICINE, vol. 146, March 1987; M.E. GEFFNER et al., pp. 337-343
- JOURNAL OF AUTOIMMUNITY, vol. 1, 1988; P.G. COLMAN et al., pp. 109-117
- EXPERIMENTAL CLINICAL ENDOCRINOLOGY, vol. 91, no. 1, 1988; H. KEILACKER et al., pp. 13-19
- IMMUNOLOGICAL INVESTIGATIONS, vol. 17, no. 3, 1988; S. SAKATA et al., pp. 237-242

## Description

This invention relates to a method for detecting the onset of insulin-dependent diabetes (IDD).

Diabetes is a chronic, complex metabolic disease that results in the inability of the body to properly maintain and use carbohydrates, fats, and proteins. It results from the interaction of various hereditary and environmental factors and is characterized by high blood glucose levels caused by a deficiency in insulin production or an impairment of its utilization. Most cases of diabetes fall into two clinical types: insulin-dependent diabetes mellitus (IDDM or IDD) and non-insulin-dependent diabetes mellitus (NIDDM or NIDD). Each type has a different prognosis, treatment, and cause.

Approximately 5 to 10 percent of diabetes patients have IDD, formerly known as juvenile diabetes because of its frequent appearance early in life, usually in childhood or adolescence. IDD is characterized by a partial or complete inability to produce insulin. Patients with IDD would die without daily insulin injections to control their disease.

Few advancements in resolving the pathogenesis of diabetes were made until the mid-1970s when evidence began to accumulate to suggest that IDD had an autoimmune etiopathogenesis. It is now generally accepted that IDD results from the chronic autoimmune destruction of the insulin producing pancreatic beta cells. Lymphocytes and other inflammatory cells have been observed within the islets of Langerhans in newly diagnosed IDD patients and have been found preferentially in regenerating islets composed of beta cells rather than those of other cell types. This active immunological process is associated with a variety of autoantibodies to beta cell cytoplasmic and membrane antigens, insulin, and insulin receptors.

Thus, IDD is a disease which is replete with autoantibodies. These include islet cell autoantibodies of the cytoplasmic type (ICA) (Gepts, W. and J. De Mey [1978] Diabetes 27(Suppl. 1):251-261); islet cell surface autoantibodies (ICSA) (Herold, K.C., A.H.J. Huen, A.H. Rubenstein, and A. Lernmark [1984] In: Immunology in Diabetes, D. Andreani, U. De Mario, K.F. Federlin, and L.G. Heding, eds., pp. 105-120, Krimpton Medical Publications, London); insulin autoantibodies (IAA) (Gorsuch, A.N., K.M. Spencer, J. lister, E. Wolf, G.F. Bottazzo, and A.G. Cudworth [1982] Diabetes 31:862-866) and the possible antiidiotypic insulin receptor autoantibodies (Ins.R.A.) (Maron, R., D. Elias, M. de J. Bartelt, G.J. Bruining, J.J. Van Rood, Y. Shechter, and I.R. Cohen [1983] Nature 303:817-818). In 1982 it was reported that antibodies to a 64,000 Mᵣ islet cell antigen were detected in IDD patients (Bækkeskov, S. et al. [1982] Nature 298:167-169). In a subsequent publication, Bækkeskov et al. reported the 64,000 Mᵣ antibodies associated with the IDD in the BB rat model (Bækkeskov, S. et al. [1984] Science 224:1348-1350). Later, a follow-up study investigated patients who were related to individuals with IDD and, thus, were known to be at risk for developing the disease (Baekkeskov, S. et al. [1987] J. Clin. Invest. 79:926-934). The results of this later study suggested that the 64,000 Mᵣ antibodies may be present in some IDD patients before the manifestation of clinical symptoms. However, in that same study, Bækkeskov et al. reported that the function of the 64,000 Mᵣ protein was unknown and that the data was inconclusive as to whether or not 64,000 Mᵣ antibodies were present before a decrease in β-cell function commenced.

Although many autoantibodies have been associated with IDD, research into identifying uniformly predictive autoantibodies has not been successful. There exists a substantial and long-felt need for an accurate means of detecting IDD in its early stages, prior to the onset of clinical symptoms and the requirement for insulin therapy.

According to the present invention, a method for detecting the onset of IDD comprises the detection, in a serum sample, of antibodies which immunoreact with a fragment, of approx 40 kd, of the 64 kd protein obtainable from islet cell preparations.

In order to ascertain whether specific entities are valid predictors of the onset of IDD, it is necessary to determine whether these entities are present before clinical symptoms of diabetes occur. This prediabetic period has been difficult to study because large numbers of high risk relatives of affected probands must be screened for circulating autoantibodies to identify the susceptible individuals, and long periods of observations are necessary to document the natural history of the beta cell failure in the disease. Therefore, animal models have been used to augment and further confirm data obtained for human tests.

The non-obese diabetic (NOD) mouse is a useful animal model for human IDD. Analysis of the NOD mouse provides important insights into the sequence of pathogenic events and leads to an understanding of the nature of the target islet cell autoantigens involved in the autoimmunological process. Another important model for IDD is the Biobreeding (BB) rat. The subject invention was discovered as a result of research and studies involving humans, BB rats, and NOD mice.

It was found that in IDD of man and the BB rat model, islet cell autoimmunities are associated with autoantibodies to a beta cell protein of relative molecular mass (Mᵣ) 64,000 (64K). It has also been determined that sera from newly-diagnosed NOD mice similarly contain an autoantibody that immunoprecipitates the 64K antigen from detergent lysates of ³⁵S methionine labelled murine islet cells (Atkinson, M., and N.K. Maclaren [1988] Diabetes 38:1587-1590). In NOD mice the autoantibody was detectable by the time of weaning, it disappeared within weeks after diabetes onset, and was absent in older non-diabetic mice as well as all of three non-diabetes-prone control strains tested.

In humans, the 64KA were present before clinical diagnosis in 96% of individuals who subsequently were under the age of 35 at IDD onset. The 64KA were found to be predictably present well in advance of the clinical manifestations of IDD. Also, the presence of 64KA was found to be highly specific to IDD; unlike IAA or ICA, 64KA has not been observed in individuals who are not at high risk, or increased risk, to develop IDD.

### Example 1

In order to confirm that the 64K Protein was indeed present within the plasma membrane, we made crude membrane preparations of islet cells, and using the reagent Triton X-114, immunoprecipitated the 64K protein using sera from patients with IDD. Next, we developed an improved method for the detection of autoantibodies to 64K protein. The method used no detergent, and as gives a result that is easier to interpret and more rapid. The method involves making crude membrane preparations of ³⁵S methionine labeled islet cells, followed by trypsinization for 60 minutes at 4°C (2 mg/ml 50 mM Tris buffer, pH 7.4). The material is then centrifuged, followed by our standard immunoprecipitation technique. Following gel electrophoresis and autoradiography, a 40K band can be observed with immunoprecipitations from diabetic sera and not in controls. As we have also shown that isolated 64K protein treated with trypsin also reveals a 40K protein, this 40K band that is immunoprecipitated directly from trypsin treated islets most likely represents the same protein. Not only is the assay for the 40K protein an improvement for simpler detection of 64K autoantibodies, it also yields a product that is much more amenable to gas phase sequence analysis using imobilon membrane. The reason for this improvement is that the 40K fragment is most likely devoid of a major hydrophobic region that has caused difficulties in our determination of N-terminal amino acid sequence of 64K protein bound to IMMOBILON™ using the Applied Biosystems gas phase sequencer. It has been established that proteins with strong hydrophobic regions solubilize with solvents used in the gas phase sequencer, and therefore represent a major technical hurdle to direct sequencing of this protein. It is also much easier to visualize the 40K protein than the 64K protein in silver staining of the SDS page gels; due to lower background staining in the 40,000 Mr range than in the 64,000 Mr range.

### Example 2

Many key regulatory proteins exist in cells as either a phosphorylated or dephosphorylated form, their steady-state levels of phosphorylation reflect the relative activities of the protein kinases and protein phosphatases that catalyze the interconversion process. Phosphorylation of serine, threonine, or occasionally tyrosine residues triggers a small conformational change in these proteins that alter their biochemical properties and biological properties. Hormones and other extracellular signals transmit information to the interior of the cell by activating transmembrane signalling systems that control the production of a relatively small number of chemical mediators termed "second messengers". These substances in turn regulate the activities of protein kinases and phophatases, and so alter the phosphorylation states of many intracellular proteins.

Although the full extent of the protein kinases within the human islet remains to be identified, we have applied the in vitro kinase assay using sera and islet cell proteins as an approach to identify any protein kinases which many have a role in the autoimmune destruction that results in IDD.

Two groups of individuals were selected for the in vitro kinase assay studies. There were 7 new onset IDD patients diagnosed according to the established National Diabetes Data Group (NDDG) criteria (National Diabetes Data Group. Diabetes. 1979; 28:1039-57), that had been referred to the University of Florida, Diabetes Clinics. We also studied 8 non-diabetic controls without any known family history of autoimmune disease. Human pancreatic islets isolated from cadaveric pancreases, were of 91.2 ± 2.3% purity, and had insulin content of 0.4 ± 0.1 mU insulin per islet. Following 48 hours of in vitro culture including the exclusive use of normal human serum, the islet cells were immunoprecipitated as follows: Immune complexes were prepared with sera from either patients with IDD or controls that were incubated with islet cell detergent extracts, followed by incubation with gamma ³²P-ATP, and the products phosphorylated in vitro were analyzed on SDS-PAGE gels. Islet cells (1000/test sera) were lysed (3 h, 4°C) in 50mM Tris buffer (2% Triton X-114, Aprotinin, PMSF, NaF). The detergent lysates were incubated with normal human serum (100 µl/1000 islets), followed by adsorption to an excess of protein A Sepharose CL-4B (Pharmacia). Aliquots containing unbound (pre-cleared) lysate were incubated with either IDD or control sera (25 µl, 18 h, 4°C). Following incubation of the immunoglobulins with the protein A Sepharose CL-4B (2 h, 4°C), the complexes were then washed 3 times with 50 mM Tris-HCl (pH 7.4) with 0.1% SDS, 1.0% Triton X-114, and 2 mM EDTA, and two times with 50mM Tris-HCl (pH 7.4). Kinase reactions were initiated by adding 10 µl of 50mM HEPES buffer (pH 7.4) containing 10 mM MnCl₂, 10 mM MgCl₂, 1% Triton X-114, and 20 µCi of adenosine (gamma-32P) 5′ triphosphate (Amersham, >5000 Ci/mmol). The precipitates were suspended and incubated for 10 minutes at 30°. Reactions were terminated by the addition of electrophoresis sample buffer and heated to 95°C for 4 minutes. The ³²P labeled products were separated by discontinuous SDS 15% polyacrylamide separating gels, followed by autoradiography from 6 to 72 hours with intensifying screens (Kodak, X-omat ARS). The IDD patients sera specifically immunoprecipitated an Mᵣ 64,000 protein, which was not observed with the control sera.

More than 65 protein kinases have been identified to date (Hanks et al. [1988] Science 241:42-52), and can be classified according to the amino acid(s) phosphorylated. Identification of phosphorylated serine, threonine, and tyrosine residues can be accomplished by isolation and analysis of the ³²P labeled protein from SDS-PAGE gels. IMMOBILONT™, a membrane of polyvinylidene difluoride to which SDS-PAGE fractionated proteins can be transferred electrophoretically, was used as a matrix for the analysis of the phosphoamino acid content of the phosphoproteins. ³²P labeled Mᵣ 64,000 protein bound to IMMOBILONT™ was acid hydrolysed and the released phosphoamino acids and phosphopeptides were analyses by two-dimensional electrophoresis. The membranes were either subjected to autoradiography directly, or to base hydrolysis (1.0 N HCl, 2 H, 55°C) prior to autoradiography. Following alignment of the autoradiograph with the imoblion membrane, the Mᵣ 64,000 containing protein was isolated (cut) from the IMMOBILON™ membrane, and subjected to acid hydrolysis (5.7 N HCl, 1 h, 110°C). The hydrolysis products were analyzed by two-dimensional paper electrophoresis after suspension in buffer containing authentic phosphotyrosine (Y), phosphoserine (S), and phosphothreonine (T) standards. Separation was performed for 4 hours at 900 volts from left (cathode) to right (anode) in acetic acid-formic acid-water (15:5:80, pH 1.9). The paper was then rotated 90° for electrophoresis in the second dimension from the bottom (cathode) to top (anode) in acetic acid-pyridine-water (5:0.5:94.5, pH 3.5) for 1.5 hours at 900 volts. The dried paper was analyzed by a BETAGEN™ analyzer, and the radioactive spots were aligned with the phosphoamino acid standards visualized by ninhydrin staining (Cooper, J. et al. [1983] Methods Enzymol. 99:387-402). Using this technique, we identified the 64,000 Mᵣ protein to have serine phosphorylation specificity; hence, a serine kinase. Because the detection of ³²P labeled phosphotyrosine containing proteins have been shown (Kamps, M. et al. [1989] Analytical Biochem. 176:22-27) to be substantially improved by incubation of the IMMOBILON™ with base prior to acid hydrolysis, parallel 2-dimensional phosphoamino acid studies using this technique were also performed. However, the base hydrolysis technique removed the ³²P signal observed in phosphoamino acid analysis of the Mᵣ 64,000 protein. This observation is again indicative of a serine-kinase specificity, and not a tyrosine specificity.

Further experimentation was performed in order to confirm the serine amino acid specificity for the phosphorylation reaction. Because of the availability of phosphotyrosine specific antisera (Boehringer Manheim, 1G2), western blots were performed (Towbin, H. et al. [1979] Proc. Natl. Acad. Sci. USA 76(9):4350-4354) using Mᵣ 64,000 protein transferred from SDS-Page gels to IMMOBILON™ membranes. Although a positive reaction was observed between the anti-phosphotyrosine antisera and a positive control phosphotyrosine, no reaction was observed between the Mᵣ 64,000 protein and the anti-pitosphotyrosine antibody.

Finally, in order to analyze if the identity of the Mᵣ 64,000 protein detected by the in vitro kinase reaction was identical to that observed with immunoprecipitates of metabolically labeled islet cells, tryptic peptide mapping experiments were performed. Treatment of the ³⁵S methionine labeled islet cell Mᵣ 64,000 protein immunoprecipitated with IDD sera showed a similar tryptic peptide mapping as that of the in vitro phosphorylated Mr 64,000 protein.

Specifically, the 64,000 Mᵣ and 125,000 Mᵣ proteins were excised from SDS-PAGE gels, and the proteins were treated with 1 mg/ml of trypsin (3-30 minutes, 4°C). The resulting proteins were re-run on SDS-PAGE gels. The 125,000 Mᵣ protein was not affected by this treatment. However, the 64,000 Mᵣ protein increased in mobility to the 40,000 Mᵣ range. Thus, this 64,000 ³²P labeled islet cell serine kinase shows the same Mᵣ (40,000) when treated with trypsin, as we have observed with the Mᵣ 64,000 ³⁵S methionine labeled islet cell protein.

## Claims

1. A method for detecting the onset of insulin-dependent diabetes, comprising the detection, in a serum sample, of antibodies which immunoreact with a fragment, of approx 40 kd, of the 64 kd protein obtainable from islet cell preparations.

2. A method according to claim 1, wherein the fragment is obtainable by trypsin digestion of the 64 kd protein.

## Patentansprüche

1. Verfahren zum Nachweis des Beginns von Insulin-abhängiger Diabetes, umfassend den Nachweis von Antikörpern in einer Serumprobe die mit einem Fragment von etwa 40 kd des aus Inselzell-Präparationen erhältlichen 64 kd-Proteins eine Immunreaktion ergeben.

2. Verfahren nach Anspruch 1, worin das Fragment durch Trypsin-Verdauung des 64 kd-Proteins erhältlich ist.

## Revendications

1. Procédé de détection de l'installation d'un diabète insulino-dépendant, comprenant la détection, dans un échantillon sérique, d'anticorps réagissant immunologiquement avec un fragment d'approximativement 40 kd de la protéine de 64 kd qu'il est possible d'obtenir à partir de préparations de cellules d'ilôts.

2. Procédé selon la revendication 1, dans lequel le fragment peut être obtenu par digestion trypsique de la protéine de 64 kd.
